# FASCICULE DE BREVET EUROPEEN

(11) **EP 0 728 459 B1**
(45) Date de publication et mention de la délivrance du brevet: **26.03.1997**
(21) Numéro de dépôt: 96400163.0
(22) Date de dépôt: 23.01.1996
(51) Int. Cl.: A61K 7/00

(54) **Composition acide à base de vésicules lipidiques et son utilisation en application topique**
Säurezusammensetzung basiert auf Lipidvesikeln sowie seiner Anwendung zur topischen Verwendung
Acidic composition based on lipid vesicles and its use in topical application

(30) Priorité: 23.02.1995 FR 9502136
(43) Date de publication de la demande: 28.08.1996
(73) Titulaire: L'OREAL, 75008 Paris (FR)
(72) Inventeur: Terren, Nadia, F-94550 Chevilly-Larue (FR); Perrin, Martine, F-91600 Savigny sur Orge (FR); Michelet, Jacques, F-91160 Champlan (FR)
(74) Mandataire: Lhoste, Catherine

(56) Documents cités:
- EP-A- 0 043 327
- EP-A- 0 347 306
- EP-A- 0 382 619
- EP-A- 0 433 132
- EP-A- 0 559 502
- EP-A- 0 582 503
- EP-A- 0 661 037
- WO-A-95/03781
- DE-C- 4 005 711
- FR-A- 2 485 921

## Description

La présente invention se rapporte à une composition de pH acide comprenant une dispersion aqueuse de vésicules lipidiques et son utilisation en application topique.

Les dispersions de vésicules lipidiques sont bien connues dans le domaine cosmétique ou en dermopharmacie.

On connaît les dispersions de vésicules lipidiques à coeur aqueux encapsulant une phase aqueuse pouvant comprendre des substances actives hydrosolubles ; lesquelles sont protégées des conditions extérieures. Ces vésicules sont obtenues à partir de lipides ioniques et/ou de lipides non-ioniques amphiphiles. Ils sont en particulier divulgués dans les demandes EP-A-0 582 503 et EP-A-0 661 037.

La demande EP-A-0 582 503 décrit des dispersions de vésicules lipidiques à coeur aqueux dont la membrane lipidique est constituées de lipides amphiphiles non-ioniques et de lipides amphiphiles ioniques neutralisés.

La demande EP-A-0 661 037 décrit des dispersions de deux types vésicules lipidiques à coeur aqueux agissant respectivement dans les couches profondes et dans les couches superficielles de l'épiderme.

On connaît également des dispersions de vésicules lipidiques à coeur huileux, encapsulant une phase huileuse pouvant comprendre des substances actives liposolubles ; lesquelles sont protégées ainsi des conditions extérieures.

Ces vésicules à coeur huileux sont décrits notamment dans les demandes de brevet français n° 93-10588 et 94-12005.

Les composés acides actifs sur le plan cosmétique ou dermatologique tels que les hydroxyacides, les α- et β-cétacides sont de plus en plus utilisés dans le domaine cosmétique pour le soin du visage et/ou du corps. En particulier, les hydroxyacides sont utilisés plus spécialement pour donner au visage un teint lumineux et éclatant et donc une bonne mine, un aspect lisse et plus jeune, pour le traitement non-thérapeutique des rides et/ou des ridules de la peau ainsi que pour faire disparaître les comédons dûs à l'acné.

Malheureusement, ces composés acides et plus particulièrement les hydroxyacides présentent l'inconvénient en raison de leur caractère acide de déstabiliser certains supports et peuvent être irritants et conduire à un important inconfort.

La demanderesse a découvert de manière surprenante que l'on pouvait réaliser des formulations acides stables en utilisant des actifs acides avec des dispersions de vésicules lipidiques . Des formulations comprenant de telles dispersions présentent, outre leur stabilité, des qualités d'application, de maquillage et de confort spécifiques aux formulations ayant pour base des systèmes vésiculaires.

Cette découverte est d'autant plus surprenante que les vésicules lipidiques à coeur huileux ou à coeur aqueux sont à priori instables en milieu acide. En effet, ils comportent une membrane lipidique susceptible de subir une hydrolysation en milieu acide.

On a constaté en effet dans la demande de brevet EP-A- 0582 503 que les dispersions acides de vésicules lipidiques à coeur aqueux dont la membrane lipidique était constituée de lipides non-ioniques amphiphiles devenaient instables lorsque la membrane comportait des composés lipidiques amphiphiles acides (lipides amphiphiles comportant au moins une fonction acide non-neutralisée) apportant l'acidité à ces dispersions. De telles dispersions pouvaient en revanche être stabilisées à un pH proche de la neutralité en incorporant dans la membrane des vésicules des lipides amphiphiles neutralisés.

On pouvait donc s'attendre à ce que la présence d'un composé acide dans ce type de dispersion vésiculaire, dans une quantité telle que le pH de la dispersion soit inférieur à 5, conduise à la détérioration des vésicules du fait qu'il soit en contact avec leur membrane lipidique.

Les compositions à base d'une dispersion de vésicules lipidiques conformes à l'invention qui comportent un composé acide dans une quantité suffisante permettant d'obtenir un pH inférieur à 5 restent, de façon inattendue, stables en milieu acide. Elles peuvent être conservées et utilisées pour la préparation de compositions à usage topique.

La présente invention a pour objet une composition comprenant une dispersion de vésicules lipidiques à coeur aqueux et/ou de vésicules lipidiques à coeur huileux, caractérisée par le fait qu'elle contient au moins un composé acide dans une quantité suffisante conférant à ladite composition un pH inférieur à 5 et que la membrane lipidique desdits vésicules à coeur aqueux ne comporte pas :
(a) de lipide amphiphile acide
(b) ni de lécithine de soja non-hydrogénée en présence de glycérine dans la dispersion.

Les compositions conformes à la présente invention, sont plus particulièrement des dispersions huile-dans-eau dans lesquelles les vésicules lipidiques agissent comme dispersant de l'huile dans la phase continue aqueuse.

Les vésicules lipidiques selon l'invention peuvent encapsuler une phase aqueuse (coeur aqueux) et/ou une phase huileuse (coeur huileux).

Une forme particulière de l'invention consiste en une composition comprenant une dispersion aqueuse de vésicules à coeur huileux en mélange avec une dispersion de vésicules à coeur aqueux encapsulant le composé acide.

Le composé acide peut être présent dans la phase aqueuse de la dispersion de vésicules à coeur aqueux ou huileux ou bien dans la phase aqueuse interne des vésicules à coeur aqueux.

Lorsque la composition est sous forme de dispersion aqueuse de vésicules à coeur huileux ou aqueux, le composé acide est introduit dans la phase aqueuse externe sous agitation, dans la dispersion de vésicules déjà formée.

Lorsque la composition est sous forme de dispersion aqueuse de vésicules à coeur huileux et de vésicules à coeur aqueux encapsulant le composé acide, le composé acide est introduit dans une dispersion de vésicules à coeur huileux déjà formée par le biais d'une dispersion de vésicules à coeur aqueux encapsulant le composé acide hydrosoluble.

Les vésicules lipidiques à coeur aqueux conformes à l'invention sont des vésicules comprenant une membrane lipidique obtenue à partir de lipides amphiphiles non-ionique, de lipides amphiphiles ioniques ou de leurs mélanges.

Ces vésicules sont décrites notamment dans la demande EP 0 504 437 et préparés selon les procédés décrits dans cette demande.

Les vésicules lipidiques à coeur huileux conformes à l'invention sont en particulier des globules huileux en dispersion aqueuse enrobés d'une couche monolamellaire ou oligolamellaire obtenue à partir d'au moins un tensioactif lipophile, un tensioactif hydrophile et soit un lipide amphiphile ionique soit un acide gras associé à un agent basique dissous dans la phase aqueuse de la dispersion.

On entend par couche "oligolamellaire" une couche comprenant de 2 à 5 lamelles lipidiques.

Ces vésicules sont décrites dans les demandes de brevet français n° 94-12005 et 93-10588.

Elles sont préparées selon un procédé consistant à mélanger dans une première étape, sous agitation, la phase huileuse comprenant l'agent tensioactif hydrophile, l'agent tensioactif lipophile, le lipide amphiphile ionique ou l'acide gras et la phase aqueuse contenant éventuellement l'agent basique, puis dans une deuxième étape, à soumettre le mélange obtenu à une homogénéisation basée sur le principe de la cavitation.

Cette homogénéisation est obtenue soit à l'aide d'ultrasons, soit à l'aide de hautes pressions comprises entre 200 et 1500 bars, soit à l'aide d'homogénéisateurs équipés d'une tète rotor-stator.

Les lipides non-ioniques amphiphiles utilisés pour la préparation des vésicules à coeur aqueux sont choisis de préférence dans le groupe formé par:
1) les éthers de polyglycérol, linéaires ou ramifiés, de formule :

   R¹⁰O-[-C₃H₅-(OH)O-]ₙ-H (II)

   dans laquelle :
   . -C₃H₅(OH)O est représenté par les structures suivantes prises en mélange ou séparément :
      -CH₂CHOHCH₂O- ; 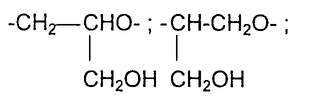
   . n est une valeur statistique moyenne comprise entre 2 et 6 ;
   . R¹⁰ représente :
      (a) une chaîne aliphatique, linéaire ou ramifiée, contenant de 12 à 18 atomes de carbone ;
      (b) un reste R¹¹CO, où R¹¹ est un radical aliphatique, linéaire ou ramifié, en C₁₁-C₁₇ ;
      (c) un reste R¹²-[-OC₂H₃(R¹³)-]-, où :
         . R¹² peut prendre la signification (a) ou (b) donnée pour R¹⁰ ;
         . OC₂H₃(R¹³)- est représenté par les structures suivantes, prises en mélange ou séparément: 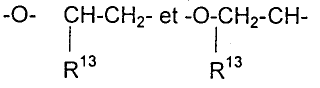 où R¹³ prend la signification (a) donnée pour R¹⁰ ;
(2) les alcools gras polyoxyéthylénés, les stérols polyoxyéthylénés ;
(3) les esters de polyols éventuellement polyoxyéthylénés ;
(4) les glycolipides naturels ou synthétiques ; et
(5) le stéarate de polyglycérol oxyéthyléné.

Les lipides ioniques amphiphiles utilisés selon l'invention pour la préparation des vésicules lipidiques à coeur huileux ou à coeur aqueux, sont choisis de préférence parmi les lipides anioniques neutralisés, les lipides amphotères et les dérivés alkylsulfoniques et plus particulièrement dans le groupe formé par :
- les sels alcalins du dicétyl et du dimyristylphosphate,
- les sels alcalins du cholestérol sulfate,
- les sels alcalins du cholestérol phosphate,
- les acylglutamates mono et disodique, et en particulier les sels mono- et disodiques de l'acide N-stéaroylglutamique ;
- les sels de sodium de l'acide phosphatidique,
- les dérivés alkylsulfoniques de formule : 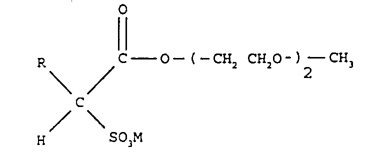
formule dans laquelle R représente les radicaux C₁₆ H₃₃ et C₁₈ H₃₇ pris en mélange ou séparément et M est un métal alcalin.
- les phosphoaminolipides ;
- les phospholipides naturels, tels que la lécithine d'oeuf ou de soja, la sphingomyéline, la phosphatidylsérine, la dipalmitoylphosphatidylcholine et les lecithines hydrogénées.

Les vésicules à coeur aqueux conformes à l'invention ont avantageusement un diamètre moyen allant de 10 à 1000 nm.

Les vésicules à coeur aqueux selon l'invention sont présents dans la composition dans des proportions allant, de préférence, de 0,5 à 15 % en poids par rapport au poids total de la composition.

Une forme particulièrement préférée de vésicules à coeur aqueux consiste en des vésicules comprenant une membrane lipidique obtenue à partir d'un mélange de stérols de soja polyoxyéthylénés et de lécithine hydrogénée.

Les agents tensioactifs lipophiles et les agents tensioactifs hydrophiles utilisés pour la préparation des vésicules à coeur huileux comportent de façon préférentielle chacun au moins une chaîne grasse saturée ayant plus de 12 atomes de carbone environ. Plus préférentiellement encore, cette chaîne grasse contient de 16 à 22 atomes de carbone.

Selon un autre mode de réalisation préférentiel de l'invention, l'agent tensioactif lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.

Des exemples de tels agents tensioactifs lipophiles sont le distéarate de sucrose, le distéarate de diglycéryle, le tristéarate de glycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de di-éthylène glycol, l'ester de glycérol et d'acides palmitique et stéarique, le monostéarate polyoxyéthyléné 2 OE (comportant 2 moles d'oxyde d'éthylène) le mono et dibéhénate de glycéryle, le tétrastéarate de pentaérythritol.

L'agent tensioactif hydrophile présente de préférence un HLB compris entre environ 8 et environ 12.

On peut citer comme exemples de tels tensioaciffs hydrophiles les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthyléné 12 OE et le distéarate de méthylglucose polyoxyéthyléné 20 OE.

L'acide gras est selon l'invention choisi de préférence parmi les acides gras saturés en C₁₆-C₂₂. On peut citer, par exemple, l'acide palmitique, l'acide stéarique, l'acide arachidique et l'acide béhénique.

L'agent basique contenu dans la phase aqueuse de la dispersion de vésicules à coeur huileux, associé à l'acide gras, est choisi par exemple parmi la soude, la triéthanolamine, la lysine ou l'arginine.

On utilise plus particulièrement des vésicules lipidiques sous forme de dispersion aqueuse de globules huileux enrobés d'une couche obtenue à partir de distéarate de saccharose, de mono-stéarate de sorbitane oxyéthyléné à 4 moles d'oxyde d'éthylène et d'un sel disodique d'acide d'acylglutamique.

Les vésicules lipidiques à coeur huileux tels que décrits ci-dessus ont de préférence une taille moyenne inférieure à 500 nanomètres et plus particulièrement inférieure à 200 nanomètres.

Les globules huileux enrobés représentent de préférence 5 à 50 % en poids par rapport au poids total de la composition.

Les compositions conformes à l'invention présentent un pH inférieur à 5 et plus particulièrement allant de 2,8 à 4.8

Les composés acides présents dans la composition de l'invention sont choisis de préférence parmi les hydroxyacides, les α- et β-cétoacides.

Les hydroxyacides utilisés conformément à la présente invention sont choisis préférentiellement parmi les α-hydroxyacides, l'acide η-octanoyl-5-salicylique ou d'acide salicylique.

On utilise plus particulièrement les α-hydroxyacides issus de fruits tels que les acides glycolique, lactique, citrique, tartrique, malique, mandélique ou leurs mélanges.

Parmi ces acides mentionnés, l'acide lactique est plus particulièrement préféré.

La quantité de composé acide présente dans les compositions de l'invention varie, de préférence, de 0,1 à 10% en poids par rapport au poids total de la composition et plus préférentiellement de 0,2 à 5% en poids et plus particulièrement de 0,5 à 4% en poids.

La phase aqueuse de la dispersion de la composition selon l'invention peut également contenir un liquide non miscible dans l'eau dispersé par les vésicules lipidiques.

Le liquide non miscible à l'eau, qui peut être présent sous forme de dispersion dans la phase aqueuse de dispersion, peut notamment être choisi dans le groupe formé par:
- les huiles animales ou végétales formées par des esters d'acide gras et de polyols, en particulier les triglycérides liquides, par exemple les huiles de tournesol, de maïs, de soja, de courge, de pépins de raisin, de jojoba, de sésame, de noisette, les huiles de poisson, le tri-caprocaprylate de glycérol, ou les huiles végétales ou animales de formule R₈COOR₉, formule dans laquelle R₈ représente le reste d'un acide gras supérieur comportant de 7 à 19 atomes de carbone et R₉ représente une chaîne hydrocarbonée ramifiée contenant de 3 à 20 atomes de carbone, par exemple l'huile de Purcellin ;
- des huiles essentielles naturelles ou synthétiques telles que, par exemple, les huiles d'euca!yptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote ;
- des hydrocarbures, tels que l'hexadécane et l'huile de paraffine ;
- des carbures halogénés, notamment des fluorocarbures tels que des fluoroamines par exemple la perfluorotributylamine, des hydrocarbures fluorés, par exemple le perfluorodécahydronaphtaléne, des fluoroesters et des fluoroéthers ;
- des silicones, par exemple les polysiloxanes, les polydiméthylsiloxanes et les fluorosilicones ;
- des esters d'acide minéral et d'un alcool ; et
- des éthers et des polyéthers.

La phase aqueuse de dispersion peut aussi également contenir des actifs cosmétiques et/ou dermopharmaceutiques, hydrosolubles. Le liquide non-miscible à l'eau peut éventuellement contenir un actif liposoluble.

La phase aqueuse de dispersion peut aussi contenir des adjuvants n'ayant ni activité cosmétique ni activité dermopharmaceutique propre, mais utilisés pour la formulation de la dispersion sous forme de lotion, crème ou sérum. Ces adjuvants sont, en particulier, pris dans le groupe formé par les gélifiants, les conservateurs, les colorants, les pigments, les opacifiants, les parfums, les filtres solaires et les poudres à usage cosmétique.

Parmi les gélifiants utilsables, on peut citer les dérivés de cellulose tels que l'hydroxyéthylcellulose, les silicates de magnésium et de sodium ou d'aluminium, les dérivés d'algues tels que le satiagum, des gommes naturelles telles que l'adragante et des polymères synthétiques, en particulier le mélange de polyacrylamide isoparaffine, alcool laurique oxyéthyléné à 7 moles d'oxyde d'éthylène vendu sous le nom SEPIGEL 305 par la Société SEPPIC.

La phase aqueuse de la dispersion peut contenir des pigments, des nanopigments, des nanotitanes enrobés ou non, des colorants solubles. Des poudres matifiantes peuvent être également ajoutées. On peut citer, par exemple, les poudres d'amidon, de nylon, les microsphères de silice, les plaquettes de mica recouvertes de microsphères de silice.

Un autre objet de l'invention consiste en des compositions destinées à être appliquées par voie topique. Elles sont caractérisées par le fait qu'elles sont constituées par une composition telle que définie précédemment.

Un autre objet de l'invention porte sur l'utilisation des compositions définies ci-dessus comme base de produits de soin ou de maquillage (crème teintée, fond de teint) pour le visage et/ou le corps.

Ces produits peuvent se présenter sous la forme de dispersions plus ou moins épaissies, de gels, de crèmes, de laits ou de sérums.

Enfin l'invention porte sur l'utilisation de la composition telle que définie précédemment pour le traitement non-thérapeutique des rides et/ou ridules de la peau et des comédons ainsi qu'un procédé de traitement cosmétique des rides et/ou des ridules de la peau ou des comédons, consistant à appliquer sur la peau la composition de l'invention.

D'autres caractéristiques et avantages de l'invention ressortiront mieux des exemples qui suivent, donnés à titre illustratif.

### EXEMPLE 1 : Fond de teint (dispersion de vésicules à coeur aqueux de pH 3,3)

| Phase A₁ : | |
|---|---|
| Stérols de soja oxyéthylénés à 5 OE non stabilisés vendus sous le nom GENEROL 122E5 | 1,6 g |
| Lécithine hydrogénée vendue sous le nom LECINOL S10 | 2,4 g |

| Phase A₂ : | |
|---|---|
| Eau déminéralisée stérile | 15 g |

| Phase A₃ : | |
|---|---|
| Eau déminéralisée stérile | 14 g |
| p-hydroxybenzoate de méthyle | 0,2 g |
| Guanosine | 0,01 g |
| Glycérine | 3 g |
| Propylèneglycol | 3 g |

| Phase B₁ : | |
|---|---|
| Huile de palme | 6,5 g |
| Huile d'amande d'abricots désodorisée (triglycérides d'acides oléique-linoléique 66/28) non stabilisée | 9,5 g |
| p-hydroxybenzoate de butyle | 0,09 g |
| p hydroxybenzoate de propyle | 0,1 g |

| Phase B₂ : | |
|---|---|
| Cyclopentadiméthylsiloxane vendue sous la dénomination VOLATILE SILICONE 7158 | 10 g |

| Phase B₃ : | |
|---|---|
| Acétate de vitamine E | 0,5 g |
| p-méthoxy-4 cinnamate d'éthyl-2 hexyle stabilisé vendu sous la dénomination PARSOL® MCX par GIVAUDAN ( filtre solaire ) | 1 g |

| Phase C : | |
|---|---|
| Oxyde de fer jaune | 0,89 g |
| Oxyde de fer brun-jaune | 0,49 g |
| Oxyde de fer noir | 0,11 g |
| Oxyde de titane (anatase) | 5,51 g |

| Phase D : | |
|---|---|
| Eau déminéralisée stérile | 1 g |
| Conservateur | 0,3 g |

| Phase E : | |
|---|---|
| Eau déminéralisée stérile | 18,84 g |
| Mélange de polyacrylamide, isoparaffine, alcool laurique oxyéthyléné (7 OE) (gélifiant) dans l'eau (35,5/23/10,5/31) vendue sous le nom SEPIGEL 305 | 2 g |

| Phase F : | |
|---|---|
| Mélange d'alphahydroxyacides issus de fruits (acide lactique/glycolique/citrique 30/15/4) dans l'eau (pH = 2) | 1 g |

| Phase G : | |
|---|---|
| Amidon réticulé par anhydride octénylsuccinique vendu sous le nom DRY FLO® | 3 g |
| Eau déminéralisée stérile qsp | 100 g |

### Mode opératoire

### I - PREPARATION DE LA PHASE VESICULAIRE A :

### Préparation de A₁ en cuve de fabrication :

On introduit l'eau de la partie A₁ à 85 ° C. On ajoute les stérols de soja oxyéthylénés (GENEROL 122 E5) sous agitation. On obtient une huile jaune et visqueuse. On ajoute la lécithine hydrogénée, sous agitation pendant 15 min. On obtient une pâte beige friable. On maintient la température à 85 ° C, sous agitation.

### Préparation de A₃ (fondoir) :

On introduit l'eau de A₃ à 85° C.

On ajoute, le propylèneglycol, la glycérine et le p-hydroxybenzoate de méthyle, sous agitation Moritz 300 t/min. On ajoute la guanosine en gardant la même agitation jusqu'à une parfaite dissolution de la guanosine.

### Mise en oeuvre de la phase vésiculaire A :

Sur A₁, on ajoute A₂ (eau) à 85° C en 3 fois sous agitation, pendant 15 minutes. On laisse le mélange A₁ + A₂ gonfler pendant 1 heure.

On introduit, par le haut de la cuve, A₃ (fondoir) à 85° C, sous agitation. On met le recyclage, sous agitation pendant 10 min. On refroidit à 55° C sous agitation.

### Passage homogénéisation haute pression.

A 55° C, on effectue 3 passages à l'homogénéisateur haute pression (500 bars). On règle l'échangeur de chaleur à surface raclée à 60° C.

Premier passage : on récupère le produit dans la cuve tampon.

Deuxième passage : on récupère le produit dans la cuve tampon.

Après le troisième passage, on transfère le produit dans la cuve de fabrication. On refroidit à 40° C sous agitation.

### II - PREPARATION DE B₁ + B₂ + B₃ (fondoir)

On introduit l'huile de palme, l'huile d'amandes d'abricots, le p-hydroxybenzoate de butyle. On chauffe à 80° C, sous agitation Moritz 500 t/min, jusqu'à homogénéisation complète. On refroidit à 65 ° C. On introduit par le fond de la cuve la silicone volatile. On ajoute l'acétate de vitamine E et le filtre solaire, sous agitation Moritz 700 t/min. On refroidit à 40° C.

### III - PREPARATION DE LA DISPERSION VESICULAIRE + HUILES

On introduit, par le haut de la cuve, la partie B (fondoir) dans la partie A (cuve de fabrication). On met le recyclage, sous agitation, pendant 10 min. On refroidit à 25° C sous agitation.

On effectue 6 passage à l'homogénéisateur haute pression (500 bars). On règle l'échangeur de chaleur à 30° C.

Après le quatrième passage, on récupère le produit dans la cuve tampon. Après le cinquième passage, on récupère le produit dans la cuve de fabrication. Après le sixième passage, le produit est récupéré dans la cuve tampon.

### IV - DISPERSION DES PIGMENTS

On transfère environ 400 kg de produit de la cuve tampon à la cuve de fabrication, on ajoute les pigments (phase C). On met le recyclage sous agitation pendant 1 heure. On vérifie la dispersion de C au microscope (il ne doit pas y avoir d'amas de pigments supérieur à 30 microns).

On transfère le reste de produit de la cuve tampon à la cuve de fabrication, sous agitation. On maintient la température à 30° C.

### V - PREPARATION DE D

On solubilise le conservateur dans l'eau à température ambiante.

### VI - PREPARATION DE E

On disperse le produit SEPIGEL 305, à température ambiante, à la défloculeuse.

### VII - AJOUT DES PHASES D, E, F, G

On ajoute D et on homogénéise sous agitation pendant 5 minutes.

On ajoute E et on homogénéise pendant 5 min sous agitation. On vérifie la bonne dispersion du gel au microscope. On ajoute le mélange d'alphahydroxyacides (phase F) puis l'amidon (G) sous agitation pendant 5 min. On refroidit à 25° C sous agitation puis on arrête l'agitation. On effectue un prélèvement pour contrôle.

La composition obtenue est stable à température ambiante après 5 mois et demi de stockage. Elle est également stable après 2 mois de stockage en étuve à 45° C.

### EXEMPLE 2 : CREME TEINTEE(dispersion de vésicules à coeur aqueux de pH 3,3)

| Phase A₁ : | |
|---|---|
| Stérols de soja oxyéthylénés à 5 OE vendus sous le nom GENEROL® 122 E5 | 1,6 g |
| Lécithine hydrogénée LECINOL S10 | 2,4 g |
| Eau déminéralisée stérile | 3 g |

| Phase A₂ : | |
|---|---|
| Eau déminéralisée stérile | 12 g |

| Phase A₃ : | |
|---|---|
| Eau déminéralisée stérile | 14 g |
| p-hydroxybenzoate de méthyle | 0,2 g |
| Guanosine | 0,01 g |
| Propylène glycol | 3 g |

| Phase A₄ : | |
|---|---|
| Glycérine | 3,0 g |
| Plancton thermal biotechnologique en dispersion aqueuse non stabilisé | 1,12 g |

| Phase C₁ : | |
|---|---|
| p-méthoxy-4 cinnamate d'éthyl 2 hexyle (PARSOL® MCX) | 4 g |
| p-hydroxybenzoate de butyle | 0,05 g |
| p-hydroxybenzoate de propyle | 0,1 g |

| Phase C₂ : | |
|---|---|
| Cyclohexadiméthylsiloxane vendue sous le nom DOW CORNING® 246 FLUID | 8 g |
| Cyclopentadiméthylsiloxane (VOLATILE SILICONE 7158) | 10 g |

| Phase C₃ : | |
|---|---|
| Palmitate de vitamine A | 0,25 g |

| Phase C₄ : | |
|---|---|
| Parfum | 0,3 g |

| Phase D : | |
|---|---|
| Eau déminéralisée stérile | 1 g |
| Conservateur | 0,3 g |

| Phase E : | |
|---|---|
| Oxyde de fer jaune | 0,37 g |
| Oxyde de fer brun-jaune | 0,34 g |
| Oxyde de fer noir | 0,09 g |
| Oxyde de titane (anatase) | 4,2 g |

| Phase F : | |
|---|---|
| Eau déminéralisée stérile | 2 g |
| D-panthénol | 1 g |

| Phase G : | |
|---|---|
| Amidon réticulé par anhydride octénylsuccinique DRY FLO® | 5 g |

| Phase H : | |
|---|---|
| Eau déminéralisée stérile | 19,47 g |

| Phase I : | |
|---|---|
| Mélange d'alphahydroxyacides issus de fruits (acide lactique/glycolique/citrique 30/15/4) dans l'eau | 1 g |

| Phase J : | |
|---|---|
| SEPIGEL 305 | 2,2 g |
| Eau déminéralisée qsp | 100 g |

La composition est préparée selon le même mode opératoire de l'exemple 1. La phase vésiculaire A (A₁ + A₂ + A₃ + A₄), le fondoir C (C₁ + C₂ + C₃ + C₄), la préparation de la dispersion vésiculaire + huiles, l'ajout des phases D, E, F, G, H, I et J sont mis en oeuvre dans les mêmes conditions définies précédemment.

La composition obtenue est stable à température ambiante après 5 mois et demi de stockage. Elle est également stable après 2 mois de stockage à 45° C.

### EXEMPLE 3 : Fond de teint(dispersion de vésicules à coeur aqueux de pH 3,3)

| Phase A₁ : | |
|---|---|
| GENEROL® 122 E5 | 1,6 g |
| LECINOL S10 | 2,4 g |

| Phase A₂ : | |
|---|---|
| Eau déminéralisée stérile | 12 g |

| Phase A₃ : | |
|---|---|
| Eau déminéralisée stérile | 14 g |
| p-hydroxy benzoate de méthyle | 0,2 g |
| Guanosine | 0,1 g |
| Propylèneglycol Glycérine | 3 g |

| Phase B₁ : | |
|---|---|
| Huile de palme | 6,5 g |
| Huile d'amandes d'abricots | 9,5 g |
| p-hydroxybenzoate de propyle | 0,1 g |
| p-hydroxybenzoate de butyle | 0,05 g |

| Phase B₂ : | |
|---|---|
| VOLATILE SILICONE 7158 | 10 g |

| Phase B₃ : | |
|---|---|
| Acétate de vitamine E | 0,5 g |
| PARSOL® MCX | 1 g |

| Phase C : | |
|---|---|
| Oxyde de fer jaune | 0,89 g |
| Oxyde de fer brun-jaune | 0,49 g |
| Oxyde de fer noir | 0,11 g |
| Oxyde de titane (anatase) | 5,51 g |

| Phase D : | |
|---|---|
| Eau déminéralisée stérile | 1 g |
| Conservateur | 0,3 g |

| Phase E : | |
|---|---|
| Eau déminéralisée stérile | 1 g |
| SEPIGEL 305 | 0,3 g |

| Phase F : | |
|---|---|
| Acide lactique dans l'eau | 1 g |

| Phase G : | |
|---|---|
| DRY FLO® | 3 g |
| Eau déminéralisée stérile qsp | 100 g |

La composition est préparée dans les mêmes conditions que celles de l'exemple 1. Elle est stable à température ambiante.

### EXEMPLE 4 : Fond de teint (Dispersion de vésicules à coeur huileux de pH 4,7)

| Phase A₁ : | |
|---|---|
| Distéarate de saccharose (tensioactif lipophile) | 2,4 g |
| Stéarate de sorbitane oxyéthyléné à 4 moles d'oxyde d'éthylène vendu par la Société ICI sous le nom TWEEN 61 (tensioactif hydrophile) | 1,6 g |
| Sel disodique de l'acide N-stéaroyl glutamique vendu par la Société AJINOMOTO sous le nom ACYLGLUTAME HS 21 (lipide amphilique ionique) | 1,2 g |
| Mélange d'huiles de tournesol, rosier, muscat, pépins de cassis (31/60/5,95/3) stabilisé | 5 g |
| Isoparaffine (6-8 moles d'isobutylène) hydrogénée | 9 g |
| Polydiméthylsiloxane vendue sous le nom DOW CORNING® FLUID 200-5CST par DOW CORNING | 9 g |
| Vitamine E (D,L-α-tocophérol) | 0,09 g |
| PARSOL® MCX | 1,0 g |

| Phase A₂ : | |
|---|---|
| Eau déminéralisée stérile | 35 g |
| Sel, disodique de l'acide éthylène diamine tétracétique (EDTA), 2H₂O Glycérine | 4 g |

| Phase B₁ : | |
|---|---|
| Eau déminéralisée stérile | 11,85 g |
| Silicate de sodium et magnésium | 0,3 g |

| Phase B₂ : | |
|---|---|
| Oxyde de fer jaune | 1,11 g |
| Oxyde de fer brun-jaune | 0,47 g |
| Oxyde de fer noir | 0,17 g |
| Oxyde de titane (anatase) | 4,25 g |
| Nano-oxyde de titane (anatase traitée par alumine/silice 4,9/0,6) | 3 g |

| Phase C : | |
|---|---|
| Eau déminéralisée stérile | 1 g |
| Conservateur | 0,3 g |

| Phase D : | |
|---|---|
| Polyéthylèneglycol à 8 moles d'oxyde éthylène | 5 g |

| Phase E : | |
|---|---|
| Poudre matifiante | 2 g |

| Phase F : | |
|---|---|
| SEPIGEL 305 | 1,2 g |

| Phase G : | |
|---|---|
| Mélange d'α-hydroxyacides : acides lactique/glycolique/citrique (30/15/4) dans l'eau | 1 g |
| Eau déminéralisée stérile qsp | 100 g |

### Mode opératoire :

### I - FABRICATION DE LA PHASE VESICULAIRE A

On fait fondre la phase A1 à 85-90° C.

On homogénéise.

En parallèle, on prépare la phase A_{*2*} (dissolution de l'EDTA) et on l'amène à 60° C.

On verse la phase A_{*2*} sur la phase A_{*1*}, rapidement et sous agitation vive. On homogénéise à 60 °C pendant 10 min. On passe l'ensemble à l'homogénéisation haute pression, sous 500 bars. 2 passages au minimum sont nécessaires. On doit obtenir un diamètre de 200 nm pour une polydispersité inférieure à 1.

On refroidit à 30° C et on désaère.

### II - PREPARATION DE LA DISPERSION PIGMENTAIRE

En parallèle, à l'aide d'un *homogène-agitateur;* on prépare le gel de silicate de magnésium et de sodium.

On introduit le gel dans la cuve de fabrication. On y disperse les pigments, les nanotitanes (phase B₂). On refroidit jusqu'à 35° C en 15 min. On introduit la phase C (conservateur).

On continue la dispersion en 1 heure et demie au total.

### III - DISPERSION DE LA DISPERSION VESICULAIRE DANS LA DISPERSION PIGMENTAIRE

On introduit en plusieurs étapes, la phase vésiculaire à 30° C sur la dispersion pigmentaire par le fond de la cuve sous agitation turbine et pâles à grande vitesse.

On laisse turbiner pendant 1 heure. On ajoute par gravité la phase D. On homogénéise pendant 5 minutes.

On ajoute la phase E (poudre) et on homogénéise pendant 2 minutes. On ajoute la phase F (gélifiant) et on homogénéise pendant 10 minutes. On désaère au maximum.

### IV - INTRODUCTION DES ALPHA-HYDROXYACIDES

A la formule ainsi obtenue sous forme gélifiée, on ajoute, sous agitation pâles vitesse rapide, la phase G. On homogénéise pendant 5 minutes.

La composition obtenue est stable à température ambiante.

### EXEMPLE 5 : Fond de teint (dispersion de vésicules à centre huileux de pH 4,7)

| Phase A₁ : | |
|---|---|
| Distéarate de saccharose | 2,4 g |
| TWEEN® 61 | 1,6 g |
| ACYLGLUTAME HS 21 | 1,2 g |
| Mélanges d'huiles de tournesol, rosier, muscat, pépins de cassis (31/60/5,95/3) | 5 g |
| Isoparaffine hydrogénée | 9 g |
| DOW CORNING® FLUID 200-5 CST | 9 g |
| Vitamine E | 0,09 g |
| PARSOL® MCX | 1 g |

| Phase A₂ : | |
|---|---|
| Eau déminéralisée stérile | 35 g |
| EDTA | 0,05 g |
| Glycérine | 4 g |

| Phase B₁ : | |
|---|---|
| Eau déminéralisée stérile | 11,85 g |
| Silicate de sodium et magnésium | 0,3 g |

| Phase B₂ : | |
|---|---|
| Oxyde de fer jaune | 1,11 g |
| Oxyde de fer brun-jaune | 0,47 g |
| Oxyde de fer noir | 0,17 g |
| Nano-oxyde de titane | 3 g |
| Oxyde de titane (anatase) | 4,25 g |

| Phase C : | |
|---|---|
| Eau déminéralisée stérile | 1 g |
| Conservateur | 0,3 g |

| Phase D : | |
|---|---|
| Polyéthylèneglycol (80 OE) | 5 g |

| Phase E : | |
|---|---|
| Poudre matifiante | 2 g |

| Phase F : | |
|---|---|
| SEPIGEL 305 | 1,2 g |

| Phase G : | |
|---|---|
| Acide lactique dans l'eau Eau déminéralisée stérile qsp | 100 g |

La composition est obtenue selon le même procédé de l'exemple 4. Elle est stable à température ambiante.

### EXEMPLE 6 : Fond de teint (dispersion de vésicules à coeur huileux et de vésicules à coeur aqueux encapsulant les α-hydroxyacides pH 4,7)

| Phase A₁ : | |
|---|---|
| Distéarate de saccharose | 1,8 g |
| TWEEN® 61 | 1,20 g |
| ACYLGLUTAMATE HS21 | 0,90 g |
| Mélange d'huiles de tournesol, rosier, muscat, pépins de cassis (31/60/5,95/3) stabilisé | 3,70 g |
| Isoparaffine hydrogénée (8 OE) | 6,70 g |
| DOW CORNING® FLUID 200.5-CST | 6,70 g |
| Vitamine E | 0,06 g |
| PARSOL® MCX | 0,70 g |

| Phase A₂ : | |
|---|---|
| Eau déminéralisée stérile | 26,12 g |
| EDTA, disodique | 0,03 g |
| Glycérine | 2,98 9 |

| Phase B₁ : | |
|---|---|
| Eau déminéralisée stérile | 7,24 g |
| Silicate de sodium et magnésium | 0,22 g |

| Phase B₂ : | |
|---|---|
| Oxyde de fer jaune | 0,83 g |
| Oxyde de fer brun-jaune | 0,35 g |
| Oxyde de fer noir | 0,13 g |
| Oxyde de titane (anatase) | 3,17 g |
| Nano-oxyde de titane de l'exemple 4 | 2,24 g |

| Phase C : | |
|---|---|
| Eau déminéralisée | 0,74 g |
| Conservateur | 0,22 g |

| Phase D : | |
|---|---|
| Polyéthylène glycol (8 OE) | 3,73 g |

| Phase E : | |
|---|---|
| Poudre matifiante | 1,49 g |

| Phase F : | |
|---|---|
| SEPIGEL 305 | 0,89 g |

| Phase G₁ : | |
|---|---|
| GENEROL® 122 E5 | 1,20 g |
| LECINOL S10 | 1,8 g |
| Eau déminéralisée stérile | 11,20 g |

| Phase G₂ : | |
|---|---|
| Eau déminéralisée stérile | 10,45 g |
| p-hydroxybenzoate de méthyle | 0,07 g |
| Propylène glycol | 2,24 g |
| Mélange d'acides lactique/glycolique/citrique (30/15/4) dans l'eau pH = 2 | 0,75 g |
| Eau déminéralisée stérile qsp | 100 g |

### Mode opératoire

La dispersion de vésicules à coeur huileux est préparée à partir des phases A₁, A₂, B₁, B₂, C, D, E et F selon les mêmes étapes du procédé de l'exemple 4 précédant l'introduction des α-hydroxyacides.

On réalise une phase vésiculaire à centre aqueux (phase G₁) dans laquelle on ajoute lors de la seconde hydratation la phase G₂ contenant les α-hydroxyacides.

Cette phase vésiculaire est alors considérée comme actif et est ajoutée, sous agitation pâles vitesse rapide à la dispersion de vésicules à coeur huileux déjà formée.

La composition obtenue est stable à température ambiante

## Revendications

1. Composition comprenant une dispersion de vésicules lipidiques à coeur aqueux et/ou de vésicules lipidiques à coeur huileux, caractérisée par le fait qu'elle contient au moins un composé acide dans une quantité suffisante conférant à ladite composition un pH inférieur à 5 et que la membrane lipidique desdits vésicules à coeur aqueux ne comporte pas :
(a) de lipide amphiphile acide
(b) ni de lécithine de soja non-hydrogénée en présence de glycérine dans la dispersion.

2. Composition selon la revendication 1, caractérisée par le fait qu'elle est une dispersion huile-dans-eau et que les vésicules lipidiques servent de dispersant de l'huile dans la phase aqueuse de la dispersion.

3. Composition selon la revendication 1 ou 2, caractérisée par le fait qu'elle comprend une dispersion de vésicules à coeur huileux en mélange avec une dispersion de vésicules à coeur aqueux encapsulant le composé acide.

4. Composition selon l'une quelconque des revendications 1 à 3, caractérisée par le fait que le composé acide est présent dans la phase aqueuse de la dispersion.

5. Composition selon l'une quelconque des revendications 1 à 4, caractérisée par le fait que le composé acide est encapsulé dans la phase aqueuse interne des vésicules à coeur aqueux.

6. Composition selon l'une quelconque des revendications 1 à 5, caractérisée par le fait que le composé acide est introduit dans la phase aqueuse de la dispersion de vésicules à coeur huileux par le biais d'une dispersion de vésicules à coeur aqueux encapsulant ledit composé acide.

7. Composition selon l'une quelconque des revendications 1 à 6, caractérisée par le fait que les vésicules à centre aqueux comprennent une membrane lipidique obtenue à partir de lipides amphiphiles non-ioniques, de lipides amphiphiles ioniques ou de leurs mélanges.

8. Composition selon l'une quelconque des revendications 1 à 7, caractérisée par le fait que les vésicules à coeur huileux sont enrobés unitairement d'une couche monolamellaire ou oligolamellaire obtenue à partir d'au moins un tensioactif lipophile, un tensioactif hydrophile et soit un lipide amphiphile ionique soit un acide gras associé à un agent basique dissous dans la phase aqueuse de la dispersion.

9. Composition selon la revendication 7 ou 8, caractérisée par le fait que les lipides amphiphiles ioniques sont choisis parmi les lipides anioniques neutralisés, les lipides amphotères et les dérivés alkylsulfoniques.

10. Composition selon la revendication 9, caractérisée par le fait que les lipides amphiphiles ioniques sont choisis dans le groupe formé par :
- les sels alcalins du dicétyl et du dimyristylphosphate,
- les sels alcalins du cholestérol sulfate,
- les sels alcalins du cholestérol phosphate,
- les acylglutamates mono et disodique,
- les sels de sodium de l'acide phosphatidique,
- les dérivés alkylsulfoniques de formule : 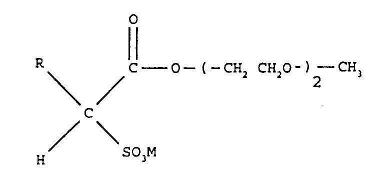
formule dans laquelle R représente les radicaux C₁₆ H₃₃ et C₁₈ H₃₇ pris en mélange ou séparément et M est un métal alcalin.
- les phosphoaminolipides,
- les phospholipides naturels.

11. Composition selon l'une quelconque des revendications 1 à 7, 9 et 10 caractérisée par le fait que les lipides amphiphiles non-ioniques sont choisis dans le groupe formé par :
1) les éthers de polyglycérol, linéaires ou ramifiés, de formule :
R¹⁰O-[-C₃H₅-(OH)O-)ₙ-H (II)
dans laquelle :
. -C₃H₅(OH)O est représenté par les structures suivantes prises en mélange ou séparément: : 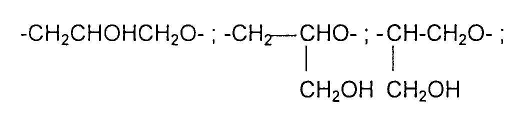
. n est une valeur statistique moyenne comprise entre 2 et 6 ;
. R¹⁰ représente :
(a) une chaîne aliphatique, linéaire ou ramifiée, contenant de 12 à 18 atomes de carbone ;
(b) un reste R¹¹CO, où R¹¹ est un radical aliphatique, linéaire ou ramifié, en C₁₁-C₁₇ ;
(c) un reste R¹²-[-OC₂H₃(R¹³)-]-, où :
. R¹² peut prendre la signification (a) ou (b) donnée pour R¹⁰ ;
. OC₂H₃(R¹³)- est représenté par les structures suivantes, prises en mélange ou séparément : 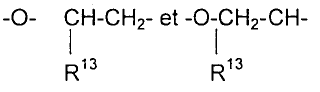 où R¹³ prend la signification (a) donnée pour R¹⁰ ;
(2) les alcools gras polyoxyéthylénés, les stérols polyoxyéthylénés ;
(3) les esters de polyols éventuellement polyoxyéthylénés ;
(4) les glycolipides naturels ou synthétiques ;
(5) le stéarate de polyglycérol oxyéthyléné.

12. Composition selon la revendication 8, caractérisée par le fait que les tensioactifs lipophiles et hydrophiles comportent une chaîne grasse ayant plus de 12 atomes de carbone.

13. Composition selon la revendication 8 ou 12, caractérisée par le fait que le tensioactif lipophile présente un HLB compris entre environ 2 et environ 5 et que le tensioactif hydrophile présente un HLB compris entre environ 8 et environ 12.

14. Composition selon la revendication 12 ou 13, caractérisée par le fait que l'agent tensioactif lipophile est choisi parmi le groupe constituant le distéarate de sucrose, le distéarate de diglycéryle, le tristéarate de tétraglycéryle, le décastéarate de décaglycéryle, le monostéarate de diglycérol, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthyléné 2 OE, (comprenant 2 moles d'oxyde d'éthylène), le mono et dibéhénate de glycéryle, le tétrastéarate de pentaérythritol.

15. Composition selon la revendication 12 ou 13, caractérisé par le fait que l'agent tensioactif hydrophile est choisi parmi les groupes comprenant le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthyléné 12 OE, le distéarate de méthylglucose polyoxyéthyléné 20 OE.

16. Composition selon l'une quelconque des revendications 8 et 12 à 15, caractérisée par le fait que l'acide gras est un alcool gras saturé en C₁₆-C₂₂.

17. Composition selon l'une quelconque des revendications 8 et 12 à 15, caractérisée par le fait que l'agent basique associé à l'acide gras est choisi parmi la soude, la triéthanolamine, la lysine ou l'arginine.

18. Composition selon l'une quelconque des revendications 1 à 7 et 9 à 10, caractérisée par le fait que les vésicules à coeur aqueux comprennent une membrane lipidique obtenue à partir de stérols de soja oxyéthylénés et de lécithine hydrogénée.

19. Composition selon l'une quelconque des revendications 8, 12 à 17, caractérisée par le fait que les vésicules à coeur huileux comprennent une membrane lipidique obtenue à partir de distéarate de saccharose, de monostéarate de sorbitane oxyéthyléné à 4 moles d'oxyde d'éthylène et d'un sel disodique d'acide d'acylglutamique.

20. Composition selon l'une quelconque des revendications 1 à 7, 9 à 11 et 18 caractérisée par le fait que les vésicules à coeur aqueux sont présents dans des proportions allant de 0,5 a 15 % en poids par rapport au poids total de la composition.

21. Composition selon l'une quelconque des revendications 1 à 8, 12 à 17 et 19, caractérisée par le fait que les vésicules à coeur huileux sont présents dans des proportions allant de 0,5 à 50 % en poids par rapport au poids total de la composition.

22. Composition selon l'une quelconque des revendications 1 à 21, caractérisée par le fait que le pH varie de 2,8 à 4,8.

23. Composition selon l'une quelconque des revendications 1 à 22, caractérisée par le fait que le composé acide est choisi parmi les hydroxyacides, les α- et β-cétoacides.

24. Composition selon la revendication 23, caractérisée par le fait que les hydroxyacides sont choisis parmi les α-hydroxyacides, l'acide salicylique ou l'acide η-octanoyl-5 salicylique.

25. Composition selon la revendication 24, caractérisée par le fait que les α-hydroxyacides sont choisis parmi les acides glycolique, tartrique, malique, citrique, mandélique, lactique ou leurs mélanges.

26. Composition selon la revendication 25, caractérisée par le fait que l'α-hydroxyacide est l'acide lactique.

27. Composition selon l'une quelconque des revendications 1 à 26, caractérisée par le fait que le composé acide est présent dans des proportions allant de 0,1 à 10% en poids par rapport au poids total de la composition.

28. Composition selon l'une quelconque des revendications 1 à 27, caractérisée par le fait qu'elle contient un liquide non-miscible dans l'eau dispersé dans la phase aqueuse de la dispersion par les vésicules lipidiques.

29. Composition selon l'une quelconque des revendications 1 à 28, caractérisée par le fait que la phase aqueuse de la dispersion contient en plus des adjuvants choisis parmi les gélifiants, les conservateurs, les pigments, les colorants, les opacifiants, les parfums, les actifs sur le plan cosmétique ou dermopharmaceutique.

30. Composition à usage topique, caractérisée par le fait qu'elle est constituée d'une composition telle que définie selon l'une quelconque des revendications 1 à 29.

31. Utilisation d'une composition selon l'une quelconque des revendications 1 à 30 comme base de produits pour le soin ou le maquillage du corps et/ou du visage.

32. Utilisation de la composition telle que définie dans l'une quelconque des revendications 1 à 30 pour le traitement non-thérapeutique des rides et/ou ridules de la peau et des comédons dûs à l'acné.

33. Procédé de traitement non-thérapeutique des ridules et/ou des rides de la peau, des comédons caractérisée par le fait qu'on applique sur la peau une composition selon l'une quelconque des revendications 1 à 30.

## Claims

1. Composition comprising a dispersion of lipid vesicles with an aqueous core and/or of lipid vesicles with an oily core, characterized in that it contains at least one acidic compound in an amount which is sufficient to impart a pH of less than 5 to the said composition and in that the lipid membrane of the said vesicles with an aqueous core does not contain:
(a) acidic amphiphilic lipids
(b) or non-hydrogenated soya lecithin in the presence of glycerol in the dispersion.

2. Composition according to Claim 1, characterized in that it is an oil-in-water dispersion and in that the lipid vesicles serve as dispersants of the oil in the aqueous phase of the dispersion.

3. Composition according to Claim 1 or 2, characterized in that it comprises a dispersion of vesicles with an oily core mixed with a dispersion of vesicles with an aqueous core encapsulating the acidic compound.

4. Composition according to any one of Claims 1 to 3, characterized in that the acidic compound is present in the aqueous phase of the dispersion.

5. Composition according to any one of Claims 1 to 4, characterized in that the acidic compound is encapsulated in the aqueous phase inside the vesicles with an aqueous core.

6. Composition according to any one of Claims 1 to 5, characterized in that the acidic compound is introduced into the aqueous phase of the dispersion of vesicles with an oily core by means of a dispersion of vesicles with an aqueous core encapsulating the said acidic compound.

7. Composition according to any one of Claims 1 to 6, characterized in that the vesicles with an aqueous centre comprise a lipid membrane obtained from nonionic amphiphilic lipids, from ionic amphiphilic lipids or from mixtures thereof.

8. Composition according to any one of Claims 1 to 7, characterized in that the vesicles with an oily core are individually coated with a monolamellar or oligolamellar layer obtained from at least one lipophilic surfactant, a hydrophilic surfactant and either an ionic amphiphilic lipid or a fatty acid combined with a basic agent dissolved in the aqueous phase of the dispersion.

9. Composition according to Claim 7 or 8, characterized in that the ionic amphiphilic lipids are chosen from neutralized anionic lipids, amphoteric lipids and alkylsulphonic derivatives.

10. Composition according to Claim 9, characterized in that the ionic amphiphilic lipids are chosen from the group formed by:
- alkaline salts of dicetyl phosphate and of dimyristyl phosphate,
- alkaline salts of cholesteryl sulphate,
- alkaline salts of cholesteryl phosphate,
- mono- and disodium acylglutamates,
- the sodium salts of phosphatidic acid,
- the alkylsulphonic derivatives of formula: 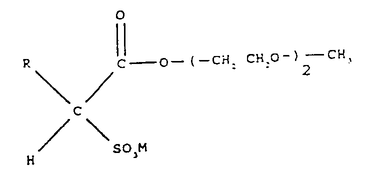
in which formula R represents the radicals C₁₆H₃₃ and C₁₈H₃₇ taken separately or as a mixture, and M is an alkali metal,
- phosphoaminolipids,
- natural phospholipids.

11. Composition according to any one of Claims 1 to 7, 9 and 10, characterized in that the nonionic amphiphilic lipids are chosen from the group formed by:
(1) linear or branched polyglycerol ethers of formula:
R¹⁰O-[-C₃H₅-(OH)O-]ₙ-H (II)
in which:
• -C₃H₅(OH)O is represented by the following structures, taken separately or as a mixture: 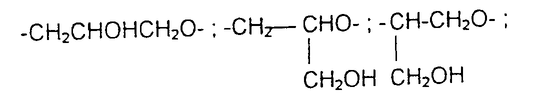
• n is an average statistical value between 2 and 6;
• R¹⁰ represents:
(a) a linear or branched aliphatic chain containing from 12 to 18 carbon atoms;
(b) a residue R¹¹CO where R¹¹ is a linear or branched C₁₁-C₁₇ aliphatic radical;
(c) a residue R¹²-[-OC₂H₃(R¹³)-]-, where:
• R¹² may take the meaning (a) or (b) given for R¹⁰;
• OC₂H₃(R¹³)- is represented by the following structures, taken separately or as a mixture: 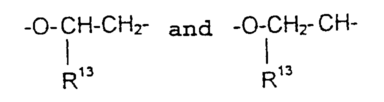 where R¹³ takes the meaning (a) given for R¹⁰;
(2) polyoxyethylenated fatty alcohols, polyoxyethylenated sterols;
(3) optionally polyoxyethylenated polyol esters;
(4) natural or synthetic glycolipids; and
(5) oxyethylenated polyglyceryl stearate.

12. Composition according to Claim 8, characterized in that the lipophilic and hydrophilic surfactants contain a fatty chain having more than 12 carbon atoms.

13. Composition according to Claim 8 or 12, characterized in that the lipophilic surfactant has an HLB of between approximately 2 and approximately 5 and in that the hydrophilic surfactant has an HLB of between approximately 8 and approximately 12.

14. Composition according to Claim 12 or 13, characterized in that the lipophilic surfactant is chosen from the group consisting of sucrose distearate, diglyceryl distearate, tetraglyceryl tristearate, decaglyceryl decastearate, diglyceryl monosteatate, hexaglyceryl tristearate, decaglyceryl pentastearate, sorbitan monostearate, sorbitan tristearate, diethylene glycol monostearate, glyceryl palmitate, glyceryl stearate, glyceryl monostearate polyoxyethylenated with 2 EO (containing 2 mol of ethylene oxide), glyceryl monobehenate, glyceryl dibehenate and pentaerythritol tetrastearate.

15. Composition according to Claim 12 or 13, characterized in that the hydrophilic surfactant is chosen from the groups comprising sorbitan monostearate polyoxyethylenated with 4 EO, sorbitan tristearate polyoxyethylenated with 20 EO, hexaglyceryl monostearate polyoxyethylenated with 8 EO, hexaglyceryl monostearate, methylglucose monostearate polyoxyethylenated with 10 EO, methylglucose distearate polyoxyethylenated with 12 EO and methylglucose distearate polyoxyethylenated with 20 EO.

16. Composition according to any one of Claims 8 and 12 to 15, characterized in that the fatty acid is a saturated C₁₆-C₂₂ fatty alcohol.

17. Composition according to any one of Claims 8 and 12 to 15, characterized in that the basic agent associated with the fatty acid is chosen from sodium hydroxide, triethanolamine, lysine and arginine.

18. Composition according to any one of Claims 1 to 7 and 9 to 10, characterized in that the vesicles with an aqueous core comprise a lipid membrane obtained from oxyethylenated soya sterols and hydrogenated lecithin.

19. Composition according to any one of Claims 8, 12 to 17, characterized in that the vesicles with an oily core comprise a lipid membrane obtained from sucrose distearate, from sorbitan monostearate oxyethylenated with 4 mol of ethylene oxide and from a disodium salt of acylglutamic acid.

20. Composition according to any one of Claims 1 to 7, 9 to 11 and 18, characterized in that the vesicles with an aqueous core are present in proportions ranging from 0.5 to 15% by weight relative to the total weight of the composition.

21. Composition according to any one of Claims 1 to 8, 12 to 17 and 19, characterized in that the vesicles with an oily core are present in proportions ranging from 0.5 to 50 % by weight relative to the total weight of the composition.

22. Composition according to any one of Claims 1 to 21, characterized in that the pH varies from 2.8 to 4.8.

23. Composition according to any one of Claims 1 to 22, characterized in that the acidic compound is chosen from hydroxy acids and α- and β-keto acids.

24. Composition according to Claim 23, characterized in that the hydroxy acids are chosen from α-hydroxy acids, salicylic acid and 5-η-octanoylsalicylic acid.

25. Composition according to Claim 24, characterized in that the α-hydroxy acids are chosen from glycolic acid, tartaric acid, malic acid, citric acid, mandelic acid and lactic acid or mixtures thereof.

26. Composition according to Claim 25, characterized in that the α-hydroxy acid is lactic acid.

27. Composition according to any one of Claims 1 to 26, characterized in that the acidic compound is present in proportions ranging from 0.1 to 10 % by weight relative to the total weight of the composition.

28. Composition according to any one of Claims 1 to 27, characterized in that it contains a water-immiscible liquid dispersed in the aqueous phase of the dispersion by the lipid vesicles.

29. Composition according to any one of Claims 1 to 28, characterized in that the aqueous phase of the dispersion also contains adjuvants chosen from gelling agents, preserving agents, pigments, dyes, opacifying agents, fragrances and cosmetically or dermopharmaceutically active agents.

30. Composition for topical use, characterized in that it consists of a composition as defined according to any one of Claims 1 to 29.

31. Use of a composition according to any one of Claims 1 to 30, as a base for body and/or facial care products or make-up.

32. Use of the composition as defined in any one of Claims 1 to 30 for the non-therapeutic treatment of wrinkles and/or fine lines on the skin and comedones due to acne.

33. Process for the non-therapeutic treatment of comedones and wrinkles and/or fine lines on the skin, characterized in that a composition according to any one of Claims 1 to 30 is applied to the skin.

## Patentansprüche

1. Zusammensetzung, die eine Dispersion von Lipidvesikeln mit wässerigem inneren Kompartiment und/oder von Lipidvesikeln mit öligem inneren Kompartiment enthält,
**dadurch gekennzeichnet, daß**
sie mindestens eine saure Verbindung in einer Menge enthält, die ausreichend ist, der Zusammensetzung einen pH-Wert unter 5 zu verleihen, und dadurch, daß die Lipidmembran der Vesikel mit wässerigem inneren Kompartiment
(a) kein saures amphiphiles Lipid und
(b) kein nicht hydriertes Sojalecithin in Gegenwart von Glycerin in der Dispersion enthält.

2. Zusammensetzung nach Anspruch 1, dadurch gekennzeichnet, daß sie eine Öl-in-Wasser-Dispersion ist und die Lipidvesikel zur Dispergierung des Öles in der wässerigen Phase der Dispersion dienen.

3. Zusammensetzung nach Anspruch 1 oder 2, dadurch gekennzeichnet, daß sie eine Dispersion von Vesikeln mit öligem inneren Kompartiment im Gemisch mit einer Dispersion von Vesikeln mit wässerigem inneren Kompartiment, in denen eine saure Verbindung eingekapselt ist, enthält.

4. Zusammensetzung nach einem der Ansprüche 1 bis 3, dadurch gekennzeichnet, daß die saure Verbindung in der wässerigen Phase der Dispersion vorliegt.

5. Zusammensetzung nach einem der Ansprüche 1 bis 4, dadurch gekennzeichnet, daß die saure Verbindung in der inneren wässerigen Phase der Vesikel mit wässerigem inneren Kompartiment eingekapselt vorliegt.

6. Zusammensetzung nach einem der Ansprüche 1 bis 5, dadurch gekennzeichnet, daß die saure Verbindung in die wässerige Phase der Dispersion von Vesikeln mit öligem inneren Kompartiment über eine Dispersion von Vesikeln mit wässerigem inneren Kompartiment, in denen die saure Verbindung eingekapselt ist, eingebracht ist.

7. Zusammensetzung nach einem der Ansprüche 1 bis 6, dadurch gekennzeichnet, daß die Vesikel mit wässerigem inneren Kompartiment eine Lipidmembran enthalten, die aus nichtionischen amphiphilen Lipiden, ionischen amphiphilen Lipiden oder deren Gemischen hergestellt ist.

8. Zusammensetzung nach einem der Ansprüche 1 bis 7, dadurch gekennzeichnet, daß die Vesikel mit öligem inneren Kompartiment einheitlich mit einer monolamellaren oder oligolamellaren Schicht umhüllt sind, die aus mindestens einem lipophilen grenzflächenaktiven Stoff, einem hydrophilen grenzflächenaktiven Stoff und entweder einem ionischen amphiphilen Lipid oder einer Fettsäure in Kombination mit einem basischen Mittel, die in der wässerigen Phase der Dispersion gelöst sind, hergestellt ist.

9. Zusammensetzung nach Anspruch 7 oder 8, dadurch gekennzeichnet, daß die ionischen amphiphilen Lipide unter den neutralisierten anionischen Lipiden, den amphoteren Lipiden und den Alkylsulfonsäurederivaten ausgewählt sind.

10. Zusammensetzung nach Anspruch 9, dadurch gekennzeichnet, daß die ionischen amphiphilen Lipide unter den folgenden Verbindungen ausgewählt sind:
- den Alkalisalzen von Dicetyl- und Dimyristylphosphat,
- den Alkalisalzen von Cholesterinsulfat,
- den Alkalisalzen von Cholesterinphosphat,
- den Mono- und Dinatriumacylglutamaten,
- den Natriumsalzen von Phosphatidsäure,
- den Alkylsulfonsäurederivaten der Formel: 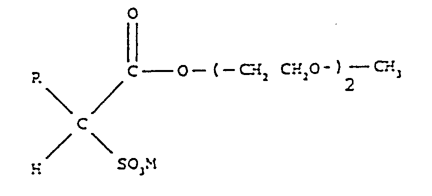
worin R die Gruppen C₁₆H₃₃ und C₁₈H₃₇ , wobei diese Gruppen im Gemisch oder einzeln vorliegen können, und M ein Alkalimetall bedeuten,
- den Phosphoaminolipiden und
- den natürlichen Phospholipiden.

11. Zusammensetzung nach einem der Ansprüche 1 bis 7, 9 und 10, dadurch gekennzeichnet, daß die nicht ionischen amphiphilen Lipide unter den folgenden Verbindungen ausgewählt sind:
(1) geradkettigen oder verzweigten Polyglycerinethern der Formel:
R¹⁰O-[-C₃H₅-(OH)O-]ₙ-H (II)
worin bedeuten:
• -C₃H₅(OH)O- die folgenden Strukturen, die im Gemisch oder einzeln vorliegen können:
-CH₂CHOHCH₂O; 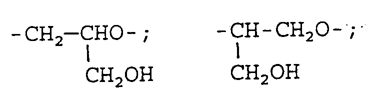
• n einen statistischen Mittelwert im Bereich von 2 bis 6;
• R¹⁰
(a) eine geradkettige oder verzweigte aliphatische Kette mit 12 bis 18 Kohlenstoffatomen;
(b) eine Gruppe R¹¹CO, wobei R¹¹ eine geradkettige oder verzweigte aliphatische Gruppe mit 11 bis 17 Kohlenstoffatomen bedeutet;
(c) eine Gruppe R¹²-[-OC₂H₃(R¹³)-]-, worin:
• R¹² die für R¹⁰ angegebenen Bedeutungen (a) oder (b) haben kann;
• OC₂H₃(R¹³) den folgenden Strukturen entspricht, wobei sie im Gemisch oder einzeln verwendet werden können, 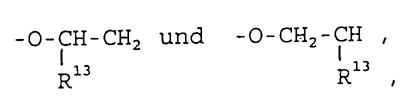 wobei R¹³ die für R¹⁰ unter (a) angegebene Bedeutung haben kann;
(2) polyethoxylierten Fettalkoholen, polyethoxylierten Sterinen;
(3) Estern von Polyolen, die gegebenenfalls polyethoxyliert sind;
(4) natürlichen oder synthetischen Glykolipiden und
(5) ethoxyliertem Polyglycerinstearat.

12. Zusammensetzung nach Anspruch 8, dadurch gekennzeichnet, daß die lipophilen und hydrophilen grenzflächenaktiven Stoffe eine Fettkette mit mehr als 12 Kohlenstoffatomen enthalten.

13. Zusammensetzung nach Anspruch 8 oder 12, dadurch gekennzeichnet, daß der lipophile grenzflächenaktive Stoff einen HLB-Wert im Bereich von etwa 2 bis etwa 5 und der hydrophile grenzflächenaktive Stoff einen HLB-Wert im Bereich von etwa 8 bis etwa 12 aufweist.

14. Zusammensetzung nach Anspruch 12 oder 13, dadurch gekennzeichnet, daß der lipophile grenzflächenaktive Stoff unter Saccharosedistearat, Diglyceryldistearat, Tetraglyceryltristearat, Dekaglyceryldecastearat, Diglycerinmonostearat, Hexaglyceryltristearat, Dekaglycerylpentastearat, Sorbitanmonostearat, Sorbitantristearat, Diethylenglykolmonostearat, Estern von Glycerin mit Palmitinsäure und Stearinsäure, Ethylenglykolmonostearat mit 2 EO (2 mol Ethylenoxid), Glycerylmono- und -dibehenat und Pentaerythrittetrastearat ausgewählt ist.

15. Zusammensetzung nach Anspruch 12 oder 13, dadurch gekennzeichnet, daß der hydrophile grenzflächenaktive Stoff unter mit 4 EO polyethoxyliertem Sorbitanmonostearat, mit 20 EO polyethoxyliertem Sorbitantristearat, Polyethylenglykolmonostearat mit 8 EO, Hexaglycerylmonostearat, Polyethylenglykolmonostearat mit 10 EO, Polyethylenglykoldistearat mit 12 EO und mit 20 EO polyethoxyliertem Methylglucosedistearat ausgewählt ist.

16. Zusammensetzung nach einem der Ansprüche 8 und 12 bis 15, dadurch gekennzeichnet, daß die Fettsäure ein gesättigter Fettalkohol mit 16 bis 22 Kohlenstoffatomen ist.

17. Zusammensetzung nach einem der Ansprüche 8 und 12 bis 15, dadurch gekennzeichnet, daß das mit der Fettsäure kombinierte basische Mittel unter Natriumhydroxid, Triethanolamin, Lysin und Arginin ausgewählt ist.

18. Zusammensetzung nach einem der Ansprüche 1 bis 7 und 9 bis 10, dadurch gekennzeichnet, daß die Vesikel mit wässerigem inneren Kompartiment eine Lipidmembran enthalten, die aus ethoxylierten Sojasterinen und hydriertem Lecithin hergestellt ist.

19. Zusammensetzung nach einem der Ansprüche 8, 12 bis 17, dadurch gekennzeichnet, daß die Vesikel mit öligem inneren Kompartiment eine Lipidmembran enthalten, die aus Saccharosedistearat, mit 4 mol Ethylenoxid ethoxyliertem Sorbitanmonostearat und einem Dinatriumsalz von Acylglutaminsäure hergestellt ist.

20. Zusammensetzung nach einem der Ansprüche 1 bis 7, 9 bis 11 und 18, dadurch gekennzeichnet, daß die Vesikel mit wässerigem inneren Kompartiment in Mengenanteilen von 0,5 bis 15 Gew.-%, bezogen auf das Gesamtgewicht der Zusammensetzung, vorliegen.

21. Zusammensetzung nach einem der Ansprüche 1 bis 8, 12 bis 17 und 19, dadurch gekennzeichnet, daß die Vesikel mit öligem inneren Kompartiment in Mengenanteilen von 0,5 bis 50 Gew.-%, bezogen auf das Gesamtgewicht der Zusammensetzung, vorliegen.

22. Zusammensetzung nach einem der Ansprüche 1 bis 21, dadurch gekennzeichnet, daß der pH-Wert im Bereich von 2,8 bis 4,8 liegt.

23. Zusammensetzung nach einem der Ansprüche 1 bis 22, dadurch gekennzeichnet, daß die saure Verbindung unter den Hydroxysäuren und den α- und β-Ketosäuren ausgewählt ist.

24. Zusammensetzung nach Anspruch 23, dadurch gekennzeichnet, daß die Hydroxysäuren unter den α-Hydroxysäuren, Salicylsäure und 5-(η-Octanoyl)-salicylsäure ausgewählt sind.

25. Zusammensetzung nach Anspruch 24, dadurch gekennzeichnet, daß die α-Hydroxysäuren unter Glykolsäure, Weinsäure, Äpfelsäure, Citronensäure, Mandelsäure, Milchsäure sowie deren Gemischen ausgewählt sind.

26. Zusammensetzung nach Anspruch 25, dadurch gekennzeichnet, daß die α-Hydroxysäure Milchsäure ist.

27. Zusammensetzung nach einem der Ansprüche 1 bis 26, dadurch gekennzeichnet, daß die saure Verbindung in Mengenanteilen von 0,1 bis 10 Gew.-%, bezogen auf das Gesamtgewicht der Zusammensetzung, vorliegt.

28. Zusammensetzung nach einem der Ansprüche 1 bis 27, dadurch gekennzeichnet, daß sie eine mit Wasser nicht mischbare Flüssigkeit enthält, die in der wässerigen Phase der Dispersion durch Lipidvesikel dispergiert ist.

29. Zusammensetzung nach einem der Ansprüche 1 bis 28, dadurch gekennzeichnet, daß die wässerige Phase der Dispersion ferner Hilfsstoffe enthält, die unter den Gelbildnern, Konservierungsmitteln, Pigmenten, Färbemitteln, Trübungsmitteln, Parfums und in der Kosmetik oder Dermopharmazie wirksamen Mitteln ausgewählt sind.

30. Zusammensetzung zur topischen Anwendung, dadurch gekennzeichnet, daß sie aus einer Zusammensetzung nach einem der Ansprüche 1 bis 29 besteht.

31. Verwendung einer Zusammensetzung nach einem der Ansprüche 1 bis 30 als Grundmasse für Produkte zur Pflege oder zum Schminken des Körpers und/oder des Gesichts.

32. Verwendung der Zusammensetzung nach einem der Ansprüche 1 bis 30 zur nichttherapeutischen Behandlung von Falten oder Fältchen der Haut und von Akne herrührenden Komedonen.

33. Verfahren zur nichttherapeutischen Behandlung von Fältchen und/oder Falten der Haut und Komedonen, dadurch gekennzeichnet, daß auf die Haut eine Zusammensetzung nach einem der Ansprüche 1 bis 30 aufgetragen wird.
